# EUROPEAN PATENT APPLICATION

(11) **EP 2 494 972 A2**
(43) Date of publication of application: **05.09.2012**
(21) Application number: 10826142.1
(22) Date of filing: 29.09.2010
(51) Int. Cl.: A61K 31/357

(54) **USE OF GAMBIEROL FOR TREATING AND/OR PREVENTING NEURODEGENERATIVE DISEASES RELATED TO TAU AND BETA-AMYLOID**

(30) Priority: 28.10.2009 ES 200930924
(71) Applicant: Universidade De Santiago De Compostela, 15782 Santiago de Compostela (ES)
(72) Inventor: BOTANA LOPEZ, Luis Miguel, E-15782 Santiago de Compostela (A Coruña) (ES); ALONSO LOPEZ, Eva, E-15782 Santiago de Compostela (A Coruña) (ES); VALE GONZALEZ, Carmen, E-15782 Santiago de Compostela (A Coruña) (ES)
(74) Representative: Pons Ariño, Angel
(86) International application number: PCT/ES2010/070634
(87) International publication number: WO 2011/051521

(57) **Abstract**

The present invention relates to the use of a compound of chemical structure (I) for producing a drug, preferably for preventing or treating pathological processes related to the tau and ss-amyloid proteins. Preferably, the compound used is gambierol

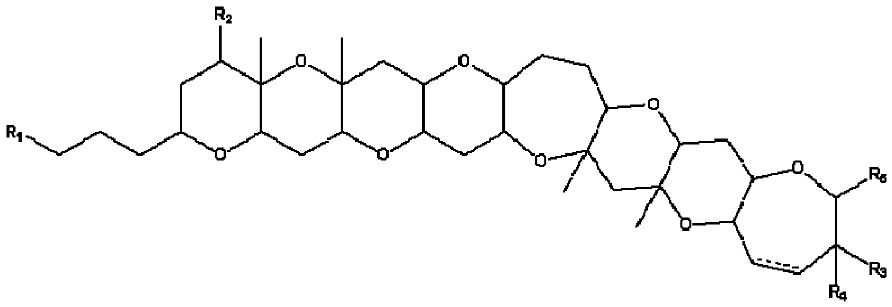

## Description

The present invention belongs to the field of biomedicine. Specifically, it relates to the use of a compound with the following chemical structure: 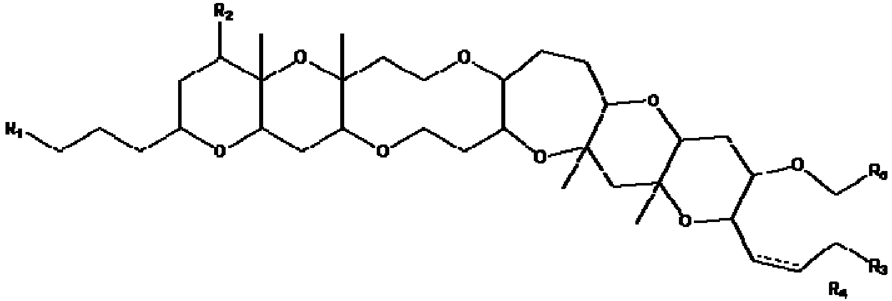 for the preparation of a medicament, preferably designed for the prevention and/or treatment of pathological processes related to the tau and β-amyloid proteins, such as, for example, Alzheimer's disease.

### STATE OF THE PRIOR ART

The treatment or prevention of neurodegenerative diseases is one of the greatest challenges faced by present-day society. These neurodegenerative diseases have an increasingly greater prevalence due to the ageing of the population. These pathologies present various etiologies, for which reason it is difficult to group them into a single classification. Nevertheless, many of them share common elements.

Amongst these diseases, the most significant, due to the fact that it is the most common cause of disability and dependency amongst people older than 65, is Alzheimer's disease (AD). This is a progressive neurodegenerative disease the origin whereof is as yet unknown. Currently, over 25 million people are afflicted by this disease worldwide, and the figure is expected to double by 2020 and to triple by 2050.

Currently, there is only one approved treatment for Alzheimer's, memantine, which is only administered at advanced stages of the disease. This compound is a non-competitive antagonist of the N-methyl-D-aspartate (NMDA) receptors, and prevents the toxic effects of the high concentrations of glutamate in the neurons. Aside from this drug, there are other compounds that delay the development of Alzheimer's, such as, for example, donepezil or rivastigmine, which act by inhibiting acetylcholinesterase (A. Fisher 2008, Neurotherapeutics; 5: 433-442).

This pathology entails the loss of patients' cognitive capacity, impairment of motor coordination and, primarily, loss of memory. This is due to the fact that there is neuronal degeneration, which degenerates into an atrophy of certain regions of the brain. At the molecular level, Alzheimer's disease is determined by the appearance of amyloid deposits, formed by the accumulation of insoluble β-amyloid, and neurofibrillary plaques due to an anomalous association of the tau protein.

Neurofibrillary plaques or tangles are intracellular structures, which accumulate in the cytoplasm of degenerated neurons. These structures have a filamentary shape. Their main component is the hyperphosphorylated tau protein, which presents an anomalous aggregation. Under non-pathological conditions, this is associated with the microtubules present in neuronal axons. These structures were initially called "paired helical filaments" or PHFs. The hyperphosphorylation of tau may be due, on the one hand, to an increase in the activity of the kinases that cause the phosphorylation of tau, and, on the other hand, to an increase in the expression, since, in this way, the substrate available for the kinases increases and, consequently, so does the product of the reaction thereof. In addition to Alzheimer's, the hyperphosphorylation of tau is involved in many other pathologies, which include, for example, Pick's disease, Parkinson's disease with dementia, frontal lobe dementia (or frontotemporal neurodegeneration) and corticobasal degeneration.

On the other hand, amyloid deposits are insoluble fibres located both intra- and extracellularly. These fibres are composed of the βA40 and βA42 forms of the β-amyloid peptide. These forms are generated by the sequential proteolytic cleavage of the β-amyloid precursor protein (APP) by β-secretases and Y-secretases (Shirwany et al. 2007 Neuropsychiatric Disease and Treatment; 3, 597-612). Under non-pathological conditions, the β-amyloid peptide is found in the brain in quantities of the order of pico or nanomolar magnitudes; for this reason, it is solubilised. An increase in the concentration of said peptide due to the anomalous processing of the APP precursor protein leads to the insolubilisation thereof and the formation of aggregates. It is well-known that certain mutations in the APP protein are related, for example, to Alzheimer's disease, since they cause the anomalous processing thereof and, consequently, the formation of β-amyloid aggregates. The β-amyloid aggregates appear in specific regions of the brain, which leads to an inflammatory response that entails neuronal death and, as a result, progressive cognitive impairment. The β-amyloid peptide is also involved in other pathologies such as, for example, neurological defects in individuals with Down syndrome or vascular dementia.

Due to the importance of both tau and β-amyloid in neurodegenerative processes, studies were initially conducted on each of the proteins independently and on the separate involvement of each one in Alzheimer's disease. As a consequence, the first approaches to the treatment of Alzheimer's were aimed at improving the effects produced by each of these proteins independently. Currently, due to the involvement of both proteins in Alzheimer's, the interactions or relations between them are being studied. These studies demonstrate the existence of a relationship between both proteins, although the mechanism thereof is as yet not well-defined. Some data show that the formation of amyloid deposits may affect different molecular pathways which may facilitate the phosphorylation of tau and, consequently, the subsequent aggregation thereof (Blurton-Jones et al. 2006, Current Alzheimer Research, 3(5), 435-448). This takes place, for example, by the activation, on the part of the β-amyloid deposits, of various kinases that include, for example, glycogen synthase 3β (GSK-3β) or cyclin-dependent kinase 5 (CDK5), which have the capacity to phosphorylate tau. Despite the existence of said relationship, the latter is not very clear, since some studies suggest that the improvement of the alteration of one of the proteins does not necessarily entail an improvement of the other, and in some cases it even makes it worse (Oddo et al., 2005. Proc Natl Acad Sci U.S.A, 102(8), 3046-51). For this reason, it is necessary to conduct studies in models that present both pathologies simultaneously.

As a consequence, it is necessary to obtain useful drugs that act jointly on the alterations of both proteins, for the prevention or treatment of diseases related to one or both alterations, such as Alzheimer's.

Currently, one of the most important sources of compounds that may be useful for the production of drugs is the marine environment. Numerous biochemical resources have been found therein which have proven to be of great medical utility, such as, for example, drugs with anti-tumour activity. Amongst these compounds, marine phycotoxins may have great clinical applicability due to their great diversity and, consequently, the numerous action mechanisms and cellular responses which they trigger. One of these compounds is gambierol, which is produced by the dinoflagellate *Gambierdiscus toxicus.* This toxin has a polycyclic ether structure, and is classified within the group of ciguatoxins (Yasumoto, 2001. Chem Rec, 1 (3), 228-242), so called due to the fact that their toxicity produces ciguatera. In the studies performed to elucidate the effects thereof, it has been determined that this toxin acts on both the voltage-dependent sodium (VGSC) and potassium (VGPC) channels. However, the administration of gambierol has not been related to the prevention or treatment of specific diseases.

### DESCRIPTION OF THE INVENTION

The present invention relates to the use of a compound with the following chemical structure (I): 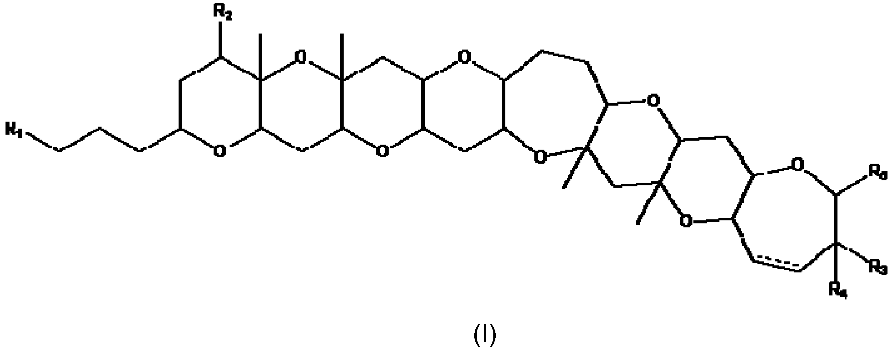 for the preparation of a medicament, preferably designed for the prevention and/or treatment of pathological processes related to tau and/or β-amyloid, such as, for example, Alzheimer's.

The examples of the present invention use *in vitro* cortical neuron cultures obtained from triple transgenic mice, which simultaneously overexpress the human transgenes for presenilin (PS1_{M146V}), the β-amyloid precursor protein (APPswe) and the tau protein (tau_{P301L}). The simultaneous overexpression of these 3 elements entails the accumulation of β-amyloid and the formation of neurofibrillary plaques, and, consequently, the *in vitro* cultures of the cells obtained from these transgenic animals are useful to study the efficacy of compounds against diseases related to increases in β-amyloid and the hyperphosphorylation of tau. The examples of the present invention demonstrate that treatment with gambierol causes a decrease in the overexpression of β-amyloid, at the intra- and extracellular levels, and a reduction of hyperphosphorylated tau at Serine residue (Ser) 202, as well as at the Threonine (Thr) 212 and Serine 214 residues.

Gambierol is a compound that belongs to the group of toxins called ciguatoxins. Its chemical structure is (II):

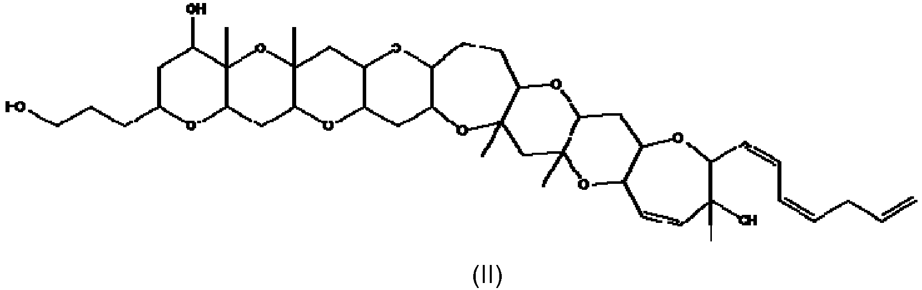

This compound exerts its toxic effects on the nervous system, specifically by inhibiting the voltage-dependent sodium (VGSC) and potassium (VGPC) channels. It has been disclosed that, in Alzheimer's disease, the potassium channels, specifically the kv3.1 channels, are diminished. Surprisingly, gambierol produces the blockage thereof, despite being useful for the reduction of both the overexpression of β-amyloid and the hyperphosphorylation of the tau protein.

On the other hand, it is worth noting that the present invention also demonstrates that the treatment of the cortical neuronal cell cultures with gambierol up to a concentration of 20 µM does not cause a reduction in the viability, despite the fact that said compound has been described as a toxin.

Therefore, the present invention provides a solution for the effective treatment of neurodegenerative diseases related to the overexpression of the β-amyloid protein and/or the hyperphosphorylation of the tau protein, which are the most relevant elements involved in the progression of diseases such as Alzheimer's.

Gambierol may present various isomers with a similar activity and functionality. Moreover, gambierol may be modified, leading to numerous derivatives that have similar physical-chemical characteristics and, consequently, a similar functionality. All these compounds present a common structure (I) with gambierol, wherein R₁, R₂ and R₃ may be identical or independently different from one another, and may be an OR' chain, a hydroxyl group or an acyl, R₄ is a methyl or a hydrogen, and R₅ may be:
- R'-O-Z, where R' is a C1-C6 alkyl, and Z is a C1-C6 alkyl or an acyl
- C1-C10 alkyl,
- C1-C10 alkenyl,
and where the symbol -̅-̅-̅-̅-̅-̅ represents a bond that may be single or double.

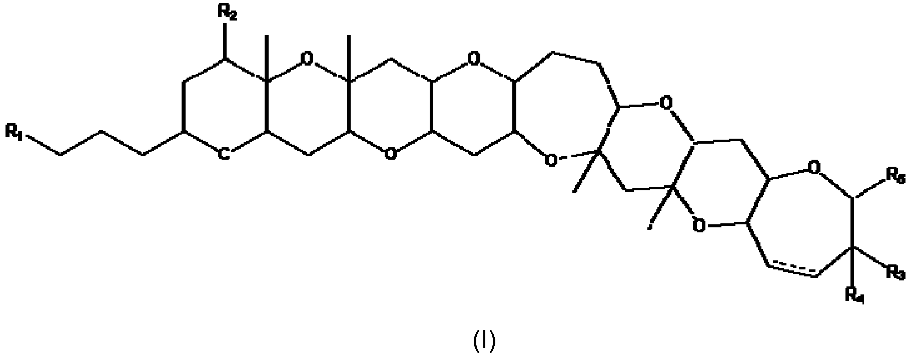

For all these reasons, a first aspect of the invention relates to the use of a compound with chemical structure (I) where:
R₁, R₂ and R₃ may be identical or independently different from one another; and may be an OR' chain, a hydroxyl or an acyl
R₄ is a methyl or a hydrogen
R₅ may be:
   - R'-O-Z, where R' is a C1-C6 alkyl, and Z is a C1-C6 alkyl or an acyl
   - C1-C10 alkyl
   - C1-C10 alkenyl, and
where the symbol -̅-̅-̅-̅-̅-̅ represents a bond that may be single or double,
for the preparation of a medicament.

A preferred embodiment of this aspect of the invention relates to the use of a compound with chemical structure (I), where R₅ is R'-O-Z, and R' is a methyl, for the preparation of a medicament.

One of the groups found in the greatest number of gambierol derivatives in an alkenyl having 7 carbon atoms in R₅, since it is one of the elements that is usually involved in the activity of these molecules. For this reason, another preferred embodiment of this aspect of the invention relates to the use of a compound with chemical structure (I), where R₅ is an alkenyl having 7 carbon atoms, for the preparation of a medicament.

In the case of gambierol, R₁, R₂ and R₃ are hydroxyl groups, whereas R₄ is a methyl group, R₅ is an alkenyl group having 7 carbon atoms and -̅-̅-̅-̅-̅-̅ is a double bond. For this reason, a more preferred embodiment of this aspect of the invention relates to the use of a compound with chemical structure (I) where R₁, R₂ and R₃ are hydroxyl groups, R₄ is a methyl group, R₅ is an alkenyl group having 7 carbon atoms, and -̅-̅-̅-̅-̅-̅ is a double bond. This compound corresponds to gambierol, which has the following chemical structure (II).

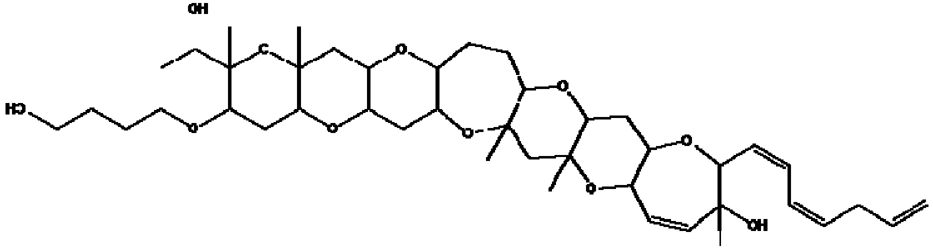

In the present invention, "alkyl" refers to linear or branched aliphatic chains, which have between 1 and 10 carbon atoms; more preferably, they have between 1 and 6 carbon atoms, for example, methyl, ethyl, n-propyl, i-propyl, n-butyl, tert-butyl, sec-butyl, n-pentyl, etc.

In the present invention, "alkenyl" refers to an alkyl radical which has between 1 and 10 carbon atoms and has one or more unsaturated bonds. The alkenyl radicals may be optionally substituted with one or more substituents, such as aryl, halogen, hydroxyl, alkoxyl, carboxyl, cyano, carbonyl, acyl, alkoxycarbonyl, amino, nitro, mercapto, etc.

In the present invention, "acyl" is understood to mean those groups derived from an oxoacid, for example, without being limited thereto, a carboxylic acid, by the elimination of at least one hydroxyl group.

The term "analogue", as used herein, refers to a chemical substance that is similar to another chemical substance in structure and/or function.

In the present invention, "derivative" is understood to mean that compound which is produced from another by means of modifications thereof, and which presents a similar functionality. These modifications may be performed, for example, without being limited thereto, by means of chemical, physical, microbiological or pharmacological methods.

Increases in both β-amyloid and the phosphorylation of tau, separately or jointly, are usually associated with pathological processes. These processes are primarily related to the nervous system, since the accumulation basically takes place in the neurons, causing the degeneration thereof. The main pathology related to these two elements jointly is Alzheimer's disease. This disease evolves with an increase in β-amyloid deposits and the hyperphosphorylation of the tau protein, which leads to neurofibrillary tangles that cause the progressive degeneration of neurons and, consequently, cognitive and motor impairment. As shown in the examples, gambierol is capable of reducing the overexpression of β-amyloid and the hyperphosphorylation of tau, but does not present any effect on any of these structures when they are not altered. This indicates that these compounds are useful for the treatment of pathologies related to an increase in the expression of β-amyloid or the hyperphosphorylation of tau both independently and jointly. For this reason, a preferred embodiment of this aspect of the invention relates to the use of a compound with chemical structure (I) or (II) for the preparation of a medicament designed for the prevention and/or treatment of neurodegenerative diseases. Another preferred embodiment of this aspect of the invention relates to the use of a compound with chemical structure (I) or (II) for the preparation of a medicament designed for the prevention and/or treatment of pathologies related to an increase in β-amyloid and/or the hyperphosphorylation of tau with respect to a control.

In the present invention, the expression "prevention and/or treatment of a pathology related to an increase in the β-amyloid protein, with respect to a control" refers to an increase in the β-amyloid protein with respect to the concentration control value of said protein. The concentration control value of the β-amyloid protein is the value of said protein in a healthy individual, i.e. an individual who does not present symptoms of any pathology related to the overproduction of the β-amyloid protein known in the state of the art. The concentration control value of the β-amyloid protein may also be the baseline value of the same individuals who suffer the disease, but when they are healthy. Said value may be an average value of the concentration levels of said protein in a group of healthy individuals. Likewise, "the hyperphosphorylation of the tau protein, with respect to a control" refers to a concentration of the tau protein that is greater than the control value of phosphorylation of said protein. The control value of phosphorylation of the tau protein is determined in the same manner described for the concentration control value of the β-amyloid protein. The concentration of the β-amyloid protein and/or the phosphorylation of the tau protein are determined in an isolated sample from an individual; preferably, the sample is a biological fluid. Preferably, the biological fluid is cephalorachidian fluid.

In the present invention, "pathology related to an increase in β-amyloid" is understood to mean all those pathologies that evolve either with an increase in the levels of the β-amyloid precursor protein or an increase in the anomalous processing of said protein, which increases the insoluble quantity and, consequently, leads to an increase in both the size and the quantity of intra- or extracellular β-amyloid deposits. These pathologies include, for example, without being limited thereto, amyotrophic lateral sclerosis, Down syndrome, vascular dementia, prion protein cerebral amyloid angiopathy and Creutzfeldt-Jacobs disease. For this reason, a preferred embodiment of this aspect of the invention relates to the use of a compound with chemical structure (I) or (II) for the preparation of a medicament designed for the prevention and/or treatment of a pathology related to an increase in β-amyloid which is selected from the list that comprises: amyotrophic lateral sclerosis, Down syndrome, vascular dementia, prion protein cerebral amyloid angiopathy and Creutzfeldt-Jacobs disease.

In the present invention, "pathology related to the hyperphosphorylation of tau" is understood to mean all those pathologies that evolve with an increase in the expression of tau, which entails an increase in the quantity of phosphorylated protein, or a hyperphosphorylation of said protein, even without any alteration in the expression, since both situations entail an increase in the size or the number of the neurofibrillary tangles produced by the anomalous aggregation of phosphorylated tau. The pathologies related to the hyperphosphorylation of tau include, for example, without being limited thereto, frontotemporal dementia, progressive supranuclear palsy, dementia associated with multiple system tauopathy, corticobasal degeneration and frontotemporal lobar degeneration or Pick's disease. For this reason, another preferred embodiment of this aspect of the invention relates to the use of a compound with chemical structure (I) or (II) for the preparation of a medicament designed for the prevention and/or treatment of a pathology related to the hyperphosphorylation of tau with respect to a control which is selected from the list that comprises: frontotemporal dementia, progressive supranuclear palsy, dementia associated with multiple system tauopathy, corticobasal degeneration and frontotemporal lobar degeneration or Pick's disease.

On the other hand, there are numerous other diseases, in addition to Alzheimer's, that evolve with simultaneous alterations in both proteins, such as, for example, without being limited thereto, moderate cognitive disorders or deficits, hereditary cerebral haemorrhage with amyloidosis of the Dutch type, cerebral amyloid angiopathy, dementia associated with Parkinson's disease, diffuse Lewy body neurodegenerative disease, corticobasal degeneration, subacute sclerosing panencephalitis, argyrophilic grain dementia and familial Gerstmann-Straussler-Scheinker disease. For this reason, another preferred embodiment of this aspect of the invention relates to the use of a compound with chemical structure (I) or (II) for the preparation of a medicament designed for the prevention and/or treatment of a pathology related to an increase in β-amyloid and the hyperphosphorylation of tau with respect to a control which is selected from the list that comprises: Alzheimer's, moderate cognitive disorders or deficits, hereditary cerebral haemorrhage with amyloidosis of the Dutch type, cerebral amyloid angiopathy, dementia associated with Parkinson's disease, diffuse Lewy body neurodegenerative disease, corticobasal degeneration, subacute sclerosing panencephalitis, argyrophilic grain dementia and familial Gerstmann-Straussler-Scheinker disease.

In the present invention, "moderate cognitive disorders or deficits" are understood to mean those alterations of a person's intellectual faculties, which include, without being limited thereto, orientation impairment, impairment of recent memory, impairment of reasoning, difficulties calculating, language problems, alteration of the capacity to perform complex tasks and alteration of the capacity to plan, which appear in the initial stages of different diseases, such as, for example, without being limited thereto, Alzheimer's, schizophrenia or senile dementia.

Any of the compounds with chemical structure (I) or (II) may be formulated jointly with other compounds to form a part of a pharmaceutical composition. In addition to this compound, the pharmaceutical composition comprises a pharmaceutically acceptable vehicle, another active principle or any combination of the compound with structure (I) or (II) with the rest of the components.

Said pharmaceutical composition may be formulated for administration to an animal, and more preferably a mammal, including a human being, in a variety of forms known in the state of the art. Thus, they may be, without being limited thereto, in aqueous or non-aqueous solutions, in emulsions or in suspensions. Examples of non-aqueous solutions are, for example, without being limited thereto, propylene glycol, polyethylene glycol, vegetable oils, such as olive oil, and injectable organic esters, such as ethyl oleate. Examples of aqueous solutions are, for example, without being limited thereto, water, alcoholic solutions in water and saline media. The aqueous solutions may or may not be buffered, and may have additional active or inactive components. The additional components include salts to modulate the ionic strength, preservatives, including, without being limited thereto, antimicrobial agents, antioxidants, chelating agents or similar, and nutrients, including glucose, dextrose, vitamins and minerals.

Alternatively, the pharmaceutical composition may be prepared for administration in solid form. The compositions may be combined with several inert vehicles or excipients, including, without being limited thereto: binding agents, such as microcrystalline cellulose, gum tragacanth or gelatin; excipients, such as starch or lactose; dispersant agents, such as alginic acid or corn starch; lubricants, such as magnesium stearate; slip agents such as colloidal silicon dioxide; sweetening agents, such as sucrose or saccharin; and flavouring agents, such as mint or methyl salicylate.

Any pharmaceutical composition described above and/or the formulations thereof may be administered to an animal, including a mammal and, therefore, a human being, in a variety of forms, including, without being limited thereto, oral, parenteral, intraperitoneal, intravenous, intradermal, epidural, intraspinal, intrastromal, intra-articular, intrasinovial, intrathecal, intralesional, intra-arterial, intracardiac, intramuscular, intranasal, intracranial, subcutaneous, intraorbital, intracapsular, topical, by means of transdermal patches, by rectal, vaginal or urethral route, by means of a suppository, percutaneous, by nasal spray, by surgical implant, by internal surgical paint, by infusion pump or by means of a catheter. A preferred embodiment of the present invention relates to the use of a compound with chemical formula (I) or (II), where said compound is administered by oral or intraperitoneal route.

The dosage to obtain a therapeutically effective quantity is dependent on a variety of factors, such as, for example, the age, weight, sex or tolerance of the mammal. In the sense used in this description, the expression "therapeutically effective quantity" refers to the quantity of the pharmaceutical composition of the invention that produces the desired effect and, in general, will be determined, amongst other causes, by the characteristics of said pharmaceutical composition and the therapeutic effect to be achieved.

Throughout the description and the claims, the word "comprises" and the variants thereof are not intended to exclude other technical characteristics, additives, components or steps. For persons skilled in the art, other objects, advantages and characteristics of the invention will arise partly from the description and partly from the practise of the invention. The following figures and examples are provided for illustrative purposes, and are not intended to limit the scope of the present invention.

### DESCRIPTION OF THE FIGURES

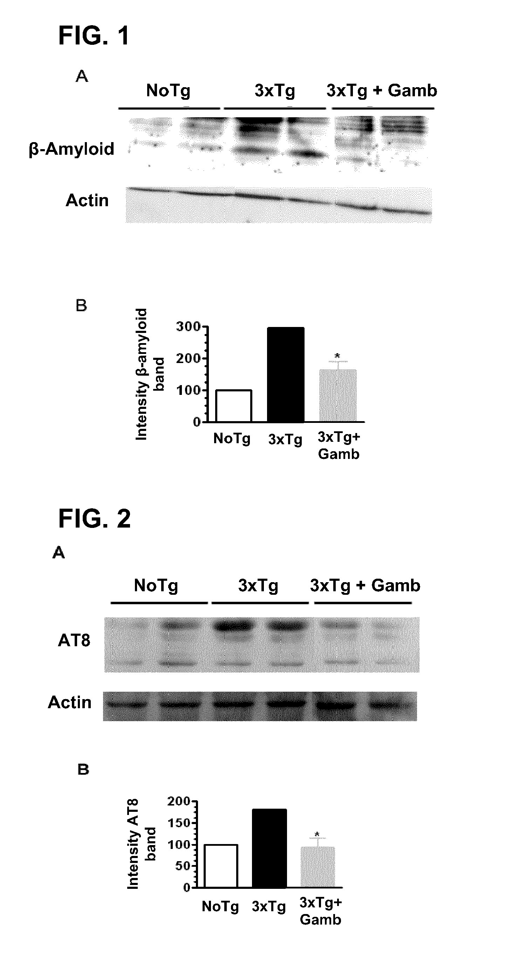

Figure 1.- Shows the decrease in the expression levels of intracellular β-amyloid following the treatment with gambierol.
   (A) Western Blot bands which represent the expression of β-amyloid in neocortical cultures from wild animals (NoTg), neocortical cultures obtained from 3xTg-AD (3xTg) mice and the levels of the β-amyloid peptide in the neocortical cultures from 3xTg-AD mice treated with gambierol (3xTg+Gamb), evaluated with the 6E10 antibody. Exposure of the cortical cultures from triple transgenic mice to gambierol reduces the overexpression of β-amyloid in this *in vitro* model. (B) The quantification of the Western Blot bands shows a significant decrease in the overexpression of β-amyloid following the treatment with gambierol (*p > 0.05 with respect to the expression of β-amyloid in transgenic cultures).
Figure 2.- Shows a decrease in the levels of phosphorylated tau in transgenic neuronal cultures treated with gambierol.
   (A) Western Blot images, which show the phosphorylation levels of tau, using the AT8 antibody (it recognises phosphorylated Tau at Ser 202), in wild cultures (NoTg), transgenic cultures (3xTg) and transgenic cultures treated with gambierol (3xTg+Gamb). (B) The quantification of the expression of phosphorylated tau (labelled with the AT8 Antibody) shows a significant decrease in the phosphorylation of tau in transgenic cultures treated with gambierol (*p < 0.05, n = 3, obtained from three representative experiments, each performed in duplicate).

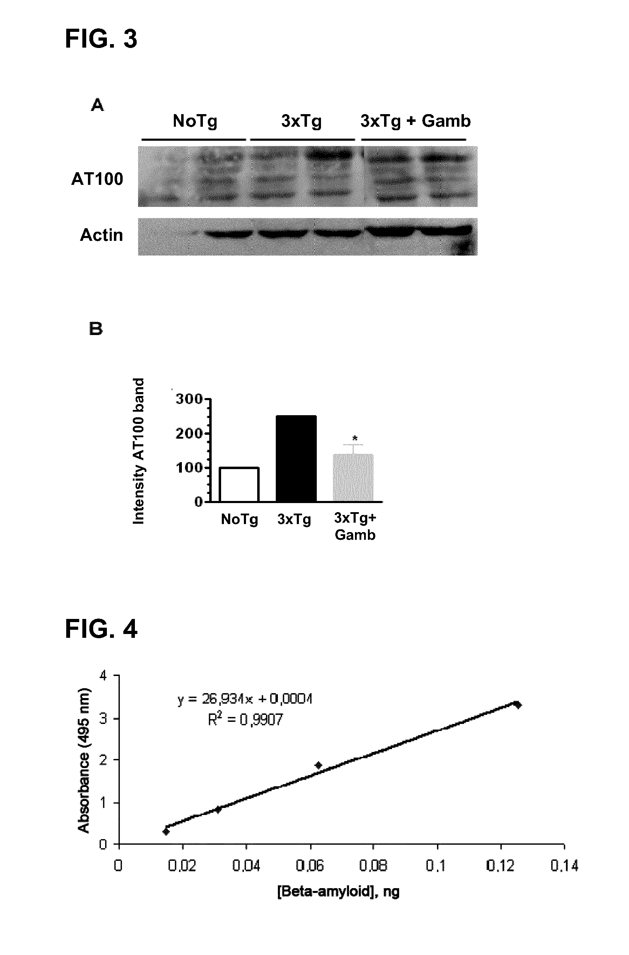

Figure 3.- Shows the inhibition of the expression of phosphorylated tau at residues Thr 212 and Ser 214 (labelled with the AT100 antibody) in transgenic neuronal cultures treated with gambierol.
   (A) Western Blot bands that show the immunoreactivity for the AT100 antibody in cultures obtained from non-transgenic mice (NoTg), transgenic cultures (3xTg) and transgenic cultures treated with gambierol (3xTg+Gamb). (B) The quantification of the expression of phosphorylated tau (labelled with the AT100 Antibody) shows a significant decrease in the phosphorylation of tau in transgenic cultures treated with gambierol (*p < 0.05, n = 3, obtained from three representative experiments, each performed in duplicate).
Figure 4.- Shows the straight-line pattern used to determine the quantity of extracellular β-amyloid peptide.
   Straight-line pattern obtained by the quantification of various concentrations of β-amyloid obtained by measuring the absorbance at a wavelength of 495 nm.
Figure 5.- Shows a decrease in the levels of extracellular β-amyloid peptide.
   These levels are determined by quantifying the β-amyloid secreted into the culture medium, in transgenic cultures treated with gambierol. Said quantification shows a decrease in the extracellular β-amyloid peptide in the triple transgenic cultures treated with gambierol as compared to those not treated. NoTg: control cultures. 3xTg: cortical neuron cultures obtained from triple transgenic animals. 3xTg+Gamb: cortical neuron cultures obtained from triple transgenic animals treated with gambierol.

### EXAMPLES

The following specific examples provided in this patent document serve to illustrate the nature of the present invention. These examples are included for illustrative purposes only and should not be interpreted as limitations to the invention claimed herein. Therefore, the examples described further below illustrate the invention without limiting the field of application thereof.

### EXAMPLE 1. Determination of the cell viability of the treatment with gambierol

In order to perform the experiments of the present invention, one of the following were used: an *in vitro* cortical neuron model with simultaneous overexpression of tau and β-amyloid obtained from an Alzheimer's disease model in triple transgenic mice (3xTg-AD or 3xTg), obtainable by means of the process explained in international application W02003/053136 and provided by the holders of said application, or an *in vitro* cortical neuron model obtained from non-transgenic mice (No Tg). The triple transgenic neuronal model presents simultaneous overexpression of the human transgenes for presenilin (PS1_{M146V}), the β-amyloid precursor protein (APP_{Swe}) and the tau protein (tau_{P301L}), which leads to an Alzheimer's model with overexpression of β-amyloid and hyperphosphorylation of tau.

The primary cortical cultures were obtained from 3xTg-AD mice embryos of 15-17 days of gestation and the wild cultures were obtained from control non-3xTg-AD mice embryos of the same strain. The effect of the compound used in this invention on the cell viability was determined by means of a fluorescence assay using the Alamar Blue viability indicator. In order to perform the assay, primary cortical cultures seeded in 96-well plates were used. The neuronal cells were incubated with the compound of the present invention, which was dissolved in dimethyl sulfoxide (DMSO) and added to the culture medium at different concentrations, and the effect thereof on the neuronal viability was determined at different times of treatment *in vitro*. The maximum concentration of gambierol evaluated *in vitro* was 20 µM and the concentration of Alamar Blue was 10%. The reaction volume used was 200 µl. The fluorescence was measured at wavelengths of 530 nm (excitation) and 590 nm (emission). The compound of the present invention did not modify the cell viability *in vitro* (Table 1).

**Table 1: Viability of cortical neurons following treatment with different concentrations of gambierol for 72 hours in culture. The data are means ± standard error of three independent experiments and are expressed as the percentage with respect to the control not treated with gambierol.**

| | **% with respect to the control** |
|---|---|
| **Control** | 100 ±5.3 |
| **0.25 µM Gambierol** | 103 ± 5.2 |
| **0.5 µM Gambierol** | 110 ± 1.4 |
| **1 µM Gambierol** | 105 ± 6.3 |
| **5 µM Gambierol** | 106 ± 4.7 |
| **10 µM Gambierol** | 105 ± 5.8 |
| **20 µM Gambierol** | 102 ± 3.8 |

### EXAMPLE 2. Effect of qambierol on the overexpression of intracellular β-amyloid and the hyperphosphorylation of tau

In order to determine the effect of the compound of the present invention on the expression of tau and β-amyloid, Western Blot techniques were used. The primary neuronal cultures were incubated with the compound of the present invention added to the culture medium for a given period of time. In the present example, the primary neuronal cultures were treated with 10 µM gambierol between days 3 and 7 of culture. Subsequently, the cells were processed following habitual Western Blot protocols. For the Western Blot studies, the protein expression was evaluated using the primary antibodies anti-β-amyloid 6E10 at a 1:500 dilution, anti-Tau AT8 (phosphorylated Tau at Ser 202, 1:1000 dilution) and anti-Tau AT100 (phosphorylated Tau at Thr 212 and Ser 214, 1:1000 dilution). The anti-β-amyloid 6E10 antibody was obtained from Covance, and the anti-tau AT100 and anti-Tau AT8 antibodies were supplied by Thermo Scientific.

For the Western Blot assays, the neuronal cultures treated with the compound of the present invention were washed with cold PBS and lysed in 50 mM Tris-HCl buffer (pH 7.4), which contains 150 mM NaCl, 1 mM EDTA, 1% Triton X-100, 2 mM DTT, 2.5 mM PMSF, 40 mg/ml aprotinin, 4 mg/ml leupeptin, 5 mM NaF, 1 mM Na3VO4, 1 mg/ml pepstatin A and 1 mg/ml benzamidine. The total protein concentration was determined by Bradford's method, using bovine albumin as the standard. Cell lysate aliquots containing a total of 20 µg of protein were loaded in loading buffer (50 mM Tris-HCl, 100 mM dithiothreitol, 2% SDS, 20% glycerol, 0.05% bromophenol blue, pH 6.8), and the proteins were separated by electrophoresis and transferred to PVDF membranes. The membranes were incubated with the primary antibodies, washed and, subsequently, incubated with an HRP-(horseradish peroxidase)-conjugated secondary antibody, and the immunoreactivity was detected by means of chemiluminescence. The same membranes were re-incubated with an anti-β-actin primary antibody in order to correct the data as a function of the protein content of the samples. These assays demonstrated that treatment with gambierol reduces the overexpression of β-amyloid (Fig. 1) and of phosphorylated tau at residue Ser 202 (Fig. 2) or residues Thr 212 and Ser 214 (Fig. 3) in neurons obtained from triple transgenic mice.

### EXAMPLE 3. Effect of the compounds of the present invention on the baseline phosphorylation of tau in control neurons

In order to determine the effect of gambierol on the baseline phosphorylation of tau, Western Blot techniques were used. An *in vitro* cortical nueron model obtained from non-transgenic mice was used. The neuronal cultures and the processing of the samples were performed in the same manner as in the preceding example. The data obtained were corrected with the quantity of β-actin as a function of the protein content of the samples. The data obtained demonstrated that treatment of the control neurons with gambierol did not affect the baseline quantity of total tau (determined by means of the Tau46 antibody, which recognises different isoforms of both phosphorylated tau and non-phosphorylated tau (Table 2), or the baseline level of phosphorylated tau at residue Ser 202 (Table 3).

**Table 2: Effect of gambierol on the baseline levels of total tau in control neurons. The data are means ± standard error of two independent experiments and are expressed as the percentage with respect to the untreated control**

| | **% with respect to the control** |
|---|---|
| **Control** | 100 ± 2.3% |
| **Control + 10 µM gambierol** | 111 ± 3.9 % |

**Table 3: Effect of gambierol on the levels of phosphorylated tau at residue Ser 202 in control neurons. The data are means ± standard error of two independent experiments and are expressed as the percentage with respect to the untreated control**

| | **% with respect to the control** |
|---|---|
| **Control** | 100 ± 4.4 % |
| **Control + 10 µM gambierol** | 107 ± 8.1% |

### EXAMPLE 4. Effect of qambierol on the overexpression of extracellular B-amyloid

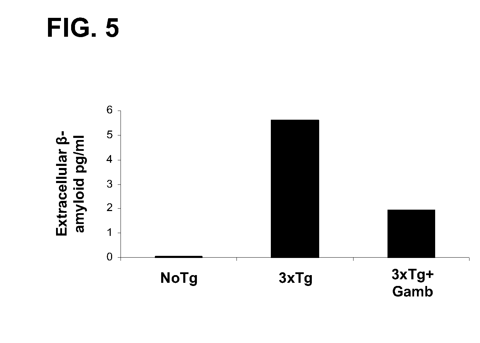

The levels of extracellular β-amyloid in the culture medium of cortical neurons obtained from triple transgenic mice or from control mice treated with gambierol were determined by means of commercial ELISA kits for the detection of β-amyloid 1-42 or β-amyloid 1-40, following the manufacturer's recommended instructions (Covance). These kits are composed of plates treated with antibodies which capture the β-amyloid peptide at the amino-terminal end thereof. The standards with known quantities of β-amyloid, and the culture medium collected from the neuronal preparations with unknown quantities of β-amyloid, were added to the wells and incubated. The plates were washed in order to eliminate the unbound peptide and incubated for 2 hours in the presence of an antibody that binds to the carboxyl-terminal end of the β-amyloid peptide. Subsequently, the plates were washed and incubated with a peroxidase-conjugated secondary antibody. At 25 minutes of incubation, the plates were washed and the substrate orto-phenylenediamine (OPD) was added in order to visualise the bound peptide. The optical density was measured by the absorbance at 495 nm, and a straight-line pattern was obtained (Fig. 4) with the quantities of β-amyloid added, wherefrom the quantity of β-amyloid in the culture medium collected from the neuronal preparations treated with the compounds of the present invention could be extrapolated (Fig. 5).

All the processes described in the present invention may be automated for HTS (high-throughput screening) using culture plates whereto the cells have been previously added, which are cultured for at least 6 days and subjected to treatment with the compounds of the present invention. These plates are susceptible to being incorporated into automatic systems that measure the markers of interest by means of different measurement methods (absorbance, luminescence, fluorescence). Likewise, the effect of the compound of the present invention, by itself or combined with other agents, on cellular, molecular and biochemical targets that are relevant in Alzheimer's and/or other neuropathological alterations which evolve with alterations in the phosphorylation of tau or the expression of β-amyloid, is susceptible to being investigated by means of HTS methods using commercial kits of interest for evaluating the effect of these treatments on the evolution of Alzheimer's disease or diseases associated with tau and β-amyloid, after the cells have been subjected to the treatment in question.

## Claims

1. Use of a compound with the following chemical structure (I): 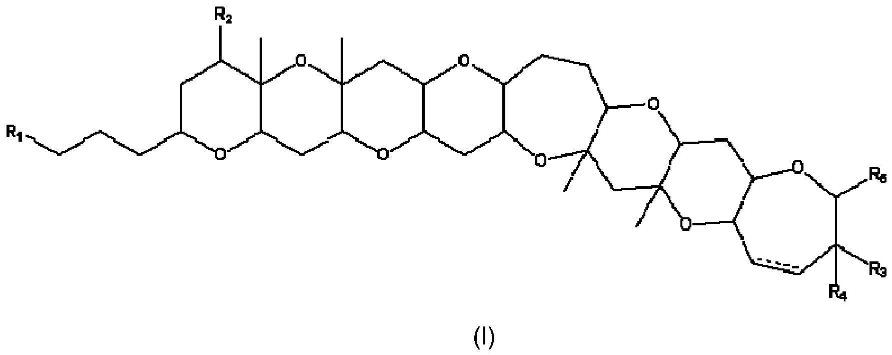 wherein:
R₁, R₂ and R₃ may be identical or independently different from one another; and may be an OR' chain, a hydroxyl or an acyl
R₄ is a methyl or a hydrogen
R₅ may be:
- R'-O-Z, where R' is a C1-C6 alkyl, and Z is a C1-C6 alkyl or an acyl
- C1-C10 alkyl
- C1-C10 alkenyl, and
the symbol -̅-̅-̅-̅-̅-̅ represents a bond that may be single or double,
for the preparation of a medicament.

2. Use of a compound according to claim 1, wherein R₅ is R'-O-Z, and R' is a methyl.

3. Use of a compound according to claim 1, wherein R₅ is an alkenyl having 7 carbon atoms.

4. Use of a compound according to claim 3, wherein R₁, R₂ and R₃ are hydroxyl groups, R₄ is a methyl group, and -̅-̅-̅-̅-̅-̅ is a double bond.

5. Use of a compound according to any of claims 1 to 4, for the preparation of a medicament for the prevention and/or treatment of a neurodegenerative disease.

6. Use of a compound according to any of claims 1 to 4, for the preparation of a medicament for the prevention and/or treatment of pathologies related to an increase in β-amyloid and/or the hyperphosphorylation of tau, with respect to a control.

7. Use of a compound according to claim 6, wherein the pathology related to an increase in β-amyloid, with respect to a control, is selected from the list that comprises: amyotrophic lateral sclerosis, Down syndrome, vascular dementia, prion protein cerebral amyloid angiopathy and Creutzfeldt-Jacobs disease.

8. Use of a compound according to claim 6, wherein the pathology related to the hyperphosphorylation of tau, with respect to a control, is selected from the list that comprises: frontotemporal dementia, progressive supranuclear palsy, dementia associated with multiple system tauopathy, corticobasal degeneration and Pick's disease.

9. Use of a compound according to claim 6, wherein the pathology related to an increase in β-amyloid and the hyperphosphorylation of tau, with respect to a control, is selected from the list that comprises: Alzheimer's disease, moderate cognitive disorders or deficits, hereditary cerebral haemorrhage with amyloidosis of the Dutch type, cerebral amyloid angiopathy, dementia associated with Parkinson's disease, diffuse Lewy body neurodegenerative disease, corticobasal degeneration, subacute sclerosing panencephalitis, argyrophilic grain dementia and familial Gerstmann-Straussler-Scheinker disease.

10. Use of a compound according to claim 9, wherein the pathology related to an increase in β-amyloid and the hyperphosphorylation of tau, with respect to a control, is Alzheimer's disease.

11. Pharmaceutical composition that comprises a compound according to any of claims 1 to 4.

12. Pharmaceutical composition according to claim 11, which further comprises a pharmaceutically acceptable vehicle.

13. Pharmaceutical composition according to any of claims 11 or 12, which further comprises another active principle.
